# EUROPEAN PATENT SPECIFICATION

(11) **EP 3 115 047 B1**
(45) Date of publication and mention of the grant of the patent: **06.05.2020**
(21) Application number: 15758693.4
(22) Date of filing: 06.03.2015
(51) Int. Cl.: A61K 31/405, A61K 31/198, A61K 9/00, A61P 3/02, A61P 21/00, A61P 25/20, A61P 25/22, A61P 25/24, A61P 25/28, A61P 43/00, A23L 2/39, A23L 2/52, A23G 1/32, A23G 1/42, A21D 2/24, A23K 20/147, A23L 33/00, A23L 33/175

(54) **DEBILITY PREVENTATIVE**
DEBILITÄTSPRÄVENTION
AGENT PRÉVENANT LA DÉBILITÉ

(30) Priority: 07.03.2014 JP 2014045247
(43) Date of publication of application: 11.01.2017
(73) Proprietor: AJINOMOTO CO., INC., Chuo-ku Tokyo 104-8315 (JP)
(72) Inventor: HAMADA, Mika, Kawasaki-shi Kanagawa 210-8681 (JP); MINE, Tomoyuki, Kawasaki-shi Kanagawa 210-8681 (JP); HAYASHI, Naoki, Kawasaki-shi Kanagawa 210-8681 (JP); UCHIDA, Ryo, Kawasaki-shi Kanagawa 210-8681 (JP)
(74) Representative: Mewburn Ellis LLP
(86) International application number: PCT/JP2015/056725
(87) International publication number: WO 2015/133627

(56) References cited:
- WO-A1-2004/026294
- WO-A1-2007/029730
- WO-A1-2013/075095
- WO-A1-2014/132985
- JP-A- H0 952 828
- JP-A- 2008 534 599
- JP-A- 2011 509 293
- JP-A- 2011 523 626
- US-A1- 2007 286 909
- SCOGNAMIGLIO, R. ET AL.: 'Oral Amino Acids in Elderly Subjects: Effect on Myocardial Function and Walking Capacity' GERONTOLOGY vol. 51, 2005, pages 302 - 308, XP008184719
- AQUILANI, R. ET AL.: 'Oral Amino Acid Supplements Improve Exercise Capacities in Elderly Patients with Chronic Heart Failure' THE AMERICAN JOURNAL OF CARDIOLOGY vol. 101, no. 11, 2008, pages S104 - S110, XP022703185

## Description

### [Technical Field]

The present invention relates to an agent for use in the prophylaxis or improvement of frailty in elderly person, which comprises isoleucine and threonine, and an agent for use in the prophylaxis or improvement of frailty in elderly person, which further comprises tryptophan and/or methionine.

### [Background Art]

Rapid aging is proceeding, and increase of health expectancy not only improves the quality of life (QOL) of elderly person, but is also desired for insurance of sustainable social life. To increase health expectancy, it is effective to prevent elderly person syndrome which is said to represent various physical and mental symptoms not necessarily considered illness, from among the symptoms that appear along with aging.

Particularly, "frailty" which is one of the elderly person syndromes is a condition before developing health problems (life dysfunction, etc.), which is generated by decrease in the spare capacity of various organs that play an important role in life activity and the ability to adapt to changes in not only the internal environment of the body but also external environment. Since prophylaxis of frailty leads to prevention or delaying of transfer to a condition in need of nursing care and the like, increases health expectancy, also improves quality of life, and decreases elderly person requiring nursing care, the prophylaxis thereof has been desired.

On the other hand, a method using muscular exercise for the prophylaxis or improvement of frailty has been reported (non-patent document 1). In muscular exercise (3 times per week) performed for person in need of nursing care (75.8±4.5 years old, n=324) as a target, the muscle mass did not increase in 3 months, and increased by 2.7% 9 months later and 5% one year later. Therefrom it is considered that exercise for a considerably long term is necessary for the recovery of muscle strength in elderly person with a small muscle mass.

Also, in a study examining the intervention effects in nutrition group, exercise (3 times per week) group, nutrition + exercise group of elderly person in residential care, who were 70 years or older, as the target for 10 weeks, the nutrition + exercise group showed the highest improvement in the muscle strength and motor function. However, it has been reported that nutrition reinforcement increases ingestion calorie and body weight, but does not improve muscle strength (non-patent document 2).

Furthermore, it is described that essential amino acid preparations accelerate recovery from physical fatigue and mental fatigue (patent publication 1), and a diet composition containing essential amino acid and not containing protein defends animals and human from aging and the like (patent publication 2). It is known that an oral amino acid-containing composition containing L-leucine can prevent or improve loss of skeletal muscle mass in elderly person (patent publication 3). However, these do not improve muscle strength or motor function.

Patent publication 4 discloses a composition comprising leucine and at least one of isoleucine, lysine, methionine, phenylalanine, threonine, tryptophan, valine, or histidine in free and/or salt form, wherein leucine in free and/or salt form is present in an amount of at least 10 to about 35% by weight based on the weight of total amino acids. The compositions are said to be suitable for promotion of muscle protein synthesis and controlling tumour-induced weight loss.

Patent publication 5 discloses compositions comprising a homogeneous mixture of free amino acids, where the free-form amino acids comprise L-lysine, L-valine, L-tryptophan, L-phenylalanine, L-methionine, L-leucine, L-threonine, L-isoleucine, L-arginine, L-histidine, L-tyrosine, L-carnitine, L-serine, L-glutamine, aspartic acid, L-proline, L-glycine, taurine, L-cysteine, gamma-aminobutyric acid (GABA), L-alanine, L-glutamic acid, and wherein the composition comprises at least one B vitamin. The compositions are said to be of use to treat disorders associated with a deficiency at least one amino acid, such as soft tissue injury, insomnia, panic attacks, anxiety disorders, eating disorders, anorexia, depression, chronic pain, emotional distress, chemical dependence, alcoholism, and hypoglycemia.

Patent publication 6 discloses a method for improving recovery of muscle strength, the method comprising administering to a subject a composition comprising the following concentrations of amino acids in terms of w/w%: about 1 to 2% of histidine, about 9 to 11 % of isoleucine, about 35 to 38% of leucine, about 14 to 17% of lysine, about 2 to 4% of methionine, about 5 to 7% of phenylalanine, about 8 to 9% of threonine, about 9 to 11 % of valine, about 0 to 0.2% of tryptophan, and about 8 to 11 % of arginine, wherein recovery of muscle strength and function of the subject is improved relative to recovery of muscle strength and function in a subject not administered said amino acid composition.

Patent publication 7 discloses a composition for increasing muscle mass, strength and functional performance, comprising leucine, carbohydrate and creatine.

Patent publication 8 discloses the use of an amino acid composition comprising at least isoleucine (Ile), tryptophan (Trp), threonine (Thr), valine (Val), histidine (His), phenylalanine (Phe), methionine (Met), lysine (Lys) and leucine (Leu) for ameliorating senile anaemia.

Non-patent document 4 discloses a study of the effect of an oral amino acid mixture on myocardial function, muscular strength and walking capacity of elderly people. The amino acid mixture is administered at the following (g/day) L-leucine 3.8, L-lysine 2, L-isoleucine 1.9, L-valine 1.9, L-threonine 1.1, L-cystine 0.4, L-histidine 0.4, L-phenylalanine 0.3, L-methionine 0.2, L-tyrosine 0.1, and L-tryptophan 0.1.

Non-patent document 5 discloses a study of oral amino acid mixture on exercise capacity of elderly patients with chronic heart failure. The mixture contains 1250 mg L-leucine, 650 mg L-lysine, 625 mg L-isoleucine, 625 mg L-valine, 350 mg L-threonine, 150 mg L-cysteine, 150 mg L-histidine, 100 mg L-phenylalanine, 50 mg L-methionine, 30 mg L-tyrosine and 20 mg L-tryptophan.

The development of an active ingredient which can improve muscle strength and motor function and prevent frailty without intervention of exercise in elderly person with a small muscle mass, and which is safe even when taken for a long term has been desired.

### [Document List]

### [Patent Documents]

patent document 1: JP-A-9-52828
patent document 2: National Publication of International Patent Application No. 2011-523626
patent document 3: National Publication of International Patent Application No. 2008-534599
patent document 4: WO 2004-026294
patent document 5: WO 2007-0286909
patent document 6: WO 2013-075095
patent document 7: WO 2009-088738
patent document 8: WO 2007-029730

### [non-patent documents]

non-patent document 1: Yamada M, et al. Effect of resistance training on physical performance and fear of falling in elderly with different levels of physical well-being. Age Ageing. 40 (5): 637-641 (2011).
non-patent document 2: Fiatarone MA, et al. Exercise training and nutritional supplementation for physical frailty in very elderly person. N Engl J Med. 330 (25): 1769-1775 (1994).
non-patent document 3: Scognamiglio R, et al. Oral amino acids in elderly subjects: effect on myocardial function and walking capacity. Gerontology 51: 302-308 (2005).
non-patent document 4: Aquilani R, et al. Oral amino acid supplements improve exercise capacities in elderly people with chronic heart failure. American Journal of Cardiology 101(11A): 104E-110E (2008).

### [SUMMARY OF THE INVENTION]

### [Problems to be Solved by the Invention]

The present invention aims to improve muscle strength, muscle mass and incentive of elderly person, and provide an agent for the prophylaxis or improvement of frailty, which can be ingested safely for a long term.

### [Means of Solving the Problems]

The present inventors have conducted intensive studies in view of the above-mentioned problems and found that a composition containing isoleucine and threonine improves various physical and mental symptoms and can prevent or improve frailty, that a composition further containing tryptophan and/or methionine can prevent or improve frailty more. Based on these findings, they have conducted further studies and completed the present invention.

Accordingly, the present invention provides the following.
[1] An agent for use in the prophylaxis or improvement of frailty, comprising isoleucine and threonine as active ingredients, wherein the content of amino acids other than tryptophan, threonine, methionine and isoleucine is not more than 60 wt% of the amino acid contained, and wherein the frailty is at least one kind of symptom selected from the group consisting of loss of muscle mass, decline in grip strength, feeling of fatigue, depression state, delirium, dementia, sleep disorder, anxiety disorder and social withdrawal, in an elderly person.
[2] The agent for use according to [1], further comprising at least one kind of amino acid selected from tryptophan and methionine as an active ingredient.
[3] The agent for use according to [1], wherein a weight ratio of tryptophan, threonine, methionine and isoleucine is 1:0.5 - 12:0.2 - 10:0.5 - 12.
[4] The agent for use according to [2] or [3], wherein the weight (%) of tryptophan, threonine, methionine and isoleucine in the free amino acid in the agent is 5% - 33%, 8% - 60%, 10% - 40% and 8% - 60% respectively.
[5] The agent for use according to any one of [1] to [4], which is in a unit package form per serving comprising not less than 0.06 g of an amino acid as an active ingredient in an ingestion amount per one serving.
[6] The agent for use according to any one of [2] to [5], which is free of an amino acid other than tryptophan, threonine, methionine and isoleucine.
[7] A food or drink for use in the prophylaxis or improvement of frailty, which is in a unit package form per serving comprising not less than 0.04 g of isoleucine and threonine in total as an active ingredient at a weight ratio of isoleucine and threonine of 1:0.2 - 10, wherein the content of amino acids other than tryptophan, threonine, methionine and isoleucine is not more than 60 wt% of the amino acid contained, and wherein the frailty is at least one kind of symptom selected from the group consisting of loss of muscle mass, decline in grip strength, feeling of fatigue, depression state, delirium, dementia, sleep disorder, anxiety disorder and social withdrawal, in an elderly person.
[8] A food or drink for use in the prophylaxis or improvement of frailty according to [7], which is in a unit package form per serving comprising not less than 0.06 g of 4 kinds of amino acids of tryptophan, threonine, methionine and isoleucine in total as an active ingredient at a weight ratio of tryptophan, threonine, methionine and isoleucine of 1:2 - 6:1 - 5:2 - 6.
[9] A kit comprising a measuring container and
   a food or drink for use in the prophylaxis or improvement of frailty, comprising isoleucine and threonine as active ingredients, wherein the content of amino acids other than tryptophan, threonine, methionine and isoleucine in the food or drink is not more than 60 wt% of the amino acid contained, and wherein the measuring container is for measuring not less than 0.04 g in total of the aforementioned amino acid, wherein the frailty is at least one kind of symptom selected from the group consisting of loss of muscle mass, decline in grip strength, feeling of fatigue, depression state, delirium, dementia, sleep disorder, anxiety disorder and social withdrawal, in an elderly person.
[10] A kit for use according to [9], comprising 4 kinds of amino acids of tryptophan, threonine, methionine and isoleucine as active ingredients, wherein the measuring container is for measuring not less than 0.06 g in total of the above-mentioned 4 kinds of amino acids.
[11] Non-therapeutic use of an agent for the prophylaxis or improvement of frailty, comprising isoleucine and threonine as active ingredients, wherein the content of amino acids other than tryptophan, threonine, methionine and isoleucine is not more than 60 wt% of the amino acid contained, and wherein the frailty is at least one kind of symptom selected from the group consisting of decrease in walking speed, decrease in the amount of physical activity and loss of motivation, in an elderly person.
[12] The non-therapeutic use according to [11], further comprising at least one kind of amino acid selected from tryptophan and methionine as an active ingredient.
[13] The non-therapeutic use according to [11] or [12], wherein a weight ratio of tryptophan, threonine, methionine and isoleucine is 1:0.5 - 12:0.2 - 10:0.5 - 12.
[14] The non-therapeutic use according to any one of [11] to [13], wherein the weight (%) of tryptophan, threonine, methionine and isoleucine in the free amino acid in the agent is 5% - 33%, 8% - 60%, 10% - 40% and 8% - 60% respectively.
[15]. The non-therapeutic use according to any one of [11] to [14], which is in a unit package form per serving comprising not less than 0.06 g of an amino acid as an active ingredient in an ingestion amount per one serving.
[16] The non-therapeutic use according to any one of [11] to [15], which is free of an amino acid other than tryptophan, threonine, methionine and isoleucine.
[17] Non-therapeutic use of a food or drink for the prophylaxis or improvement of frailty, which is in a unit package form per serving comprising not less than 0.04 g of isoleucine and threonine in total as an active ingredient at a weight ratio of isoleucine and threonine of 1:0.2 - 10, wherein the content of amino acids other than tryptophan, threonine, methionine and isoleucine is not more than 60 wt% of the amino acid contained, and wherein the frailty is at least one kind of symptom selected from the group consisting of decrease in walking speed, decrease in the amount of physical activity and loss of motivation, in elderly person.
[18] The non-therapeutic use according to [17], which is in a unit package form per serving comprising not less than 0.06 g of 4 kinds of amino acids of tryptophan, threonine, methionine and isoleucine in total as an active ingredient at a weight ratio of tryptophan, threonine, methionine and isoleucine of 1:2 - 6:1 - 5:2 - 6.

### [Effect of the Invention]

According to the present invention, muscle strength and muscle mass increase in elderly person without intervention of exercise, frailty can be prevented, and the QOL of elderly person can be enhanced by merely ingesting 2 - 4 kinds of amino acids of isoleucine and threonine, and further, tryptophan and/or methionine in the amounts specified in the claims.

According to the present invention, incentives for activity are promoted, the amount of motor activity is increased, and muscle strength and muscle mass can be increased in elderly person by merely ingesting the aforementioned 2 - 4 kinds of particular amino acids in the amounts specified in the claims.

According to the present invention, moreover, health problems of elderly person can be delayed, health expectancy can be extended, and the medical expenses and care costs can be suppressed.

Since amino acid is an active ingredient, frailty can be prevented by safely administering for a long term.

### [Brief Description of the Drawings]

Fig. 1 shows a feed intake schedule for rat.
Fig. 2 shows an influence of tryptophan (Trp), threonine (Thr), methionine (Met) and isoleucine (Ile) (hereinafter to be also referred to as 4AA) on feed intake in low protein model rats.
Fig. 3 shows an influence of 4AA on spontaneous activity of low protein model rat.
Fig. 4 shows an influence of the addition of 4AA, or 4AA excluding Trp, Thr or Met on the body weight of low protein model rat.
Fig. 5 shows an influence of 4AA, Ile and Thr, Ile or Thr on the body weight of low protein model rat.
Fig. 6 shows an influence of the addition of 4AA; essential and non-essential amino acids excluding 4AA (ALL-4AA); essential amino acid excluding 4AA (EAA-4AA), non-essential amino acid (NEAA); glycine (Gly) on the body weight of low protein model rat.
Fig. 7 shows an influence of the addition of 4AA; essential and non-essential amino acids excluding 4AA (ALL-4AA); essential amino acid excluding 4AA (EAA-4AA), non-essential amino acid (NEAA); glycine (Gly) on the weight of viscera-isolated carcass of low protein model rat.

### [Description of Embodiments]

The present invention relates to an agent for use in the prophylaxis or improvement of frailty, comprising, as active ingredients, at least isoleucine and threonine, and further, tryptophan or methionine, and an agent for the prophylaxis or improvement of frailty, comprising 4 kinds of amino acids of (1) tryptophan, (2) threonine, (3) methionine and (4) isoleucine (hereinafter an agent for use in the prophylaxis or improvement of frailty containing at least isoleucine and threonine as active ingredients is sometimes to be generically abbreviated as the agent for use in the prophylaxis or improvement of frailty of the present invention).

While the amino acids of the above-mentioned (1) - (4) to be used in the present invention may be any of L-form, D-form and DL-form, L-form is preferable.

The amino acids (1) - (4) may be salts, and examples of the salt of the amino acid include acid addition salt, metal salt, ammonium salt, organic amine addition salt, and amino acid addition salt .

Examples of the acid addition salt include inorganic acid salts such as hydrochloride, sulfate, nitrate, and phosphate,
and organic acid salts such as acetate, maleate, fumarate, citrate, malate, lactate, α-ketoglutarate, gluconate, and caprylate.

Examples of the metal salt include alkali metal salts such as sodium salt and potassium salt, alkaline earth metal salts such as magnesium salt and calcium salt, aluminum salt, and zinc salt.

Examples of the ammonium salt include salts of ammonium and tetramethylammonium .

Examples of the organic amine addition salt include salts with morpholine and piperidine.

Examples of the amino acid addition salt include salts with glycine, phenylalanine, lysine, aspartic acid and glutamic acid .

The salts of the amino acids (1) - (4) may be a hydrate (hydrate salt), and examples of such hydrate include 1 - 6 hydrates .

The amino acids (1) - (4) in the present invention may be produced by any production method such as a protein hydrolysis method, a chemical synthesis method, an enzyme method, or a fermentation method, and commercially available products can also be used.

The amino acids (1) - (4) in the present invention can also be obtained by enzymatically hydrolyzing a natural protein having a sequence containing the amino acid.

As used herein, "frailty" is one of the elderly person syndromes among the symptoms that appear along with aging, which is said to represent various physical and mental symptoms not necessarily considered illness. It refers to a "condition before developing health problems (life dysfunction, etc.), which is generated by a variety of factors involved in elderly generation that reduce the spare capacity of various organs playing an important role in life activity, and reduce the ability to adapt to changes in not only the internal environment of the body but also external environment, as a result of which physical, mental, social functions are gradually lost".

Examples of the index or specific physical symptoms of frailty include body weight decrease, loss of muscle mass (shrinkage of body), decline in grip strength (faintness), feeling of fatigue (fatiguability), decrease in walking speed (slow movement), reduced amount of physical activity (less movement), in elderly person. When at least one of these symptoms is found, frailty is judged to be present. Among others, loss of muscle mass, decline in grip strength, feeling of fatigue, decrease in walking speed and decrease in the amount of physical activity, in elderly person are preferable targets of the agent for use in the prophylaxis or improvement of frailty of the present invention.

Specific examples of the mental symptom of frailty include loss of motivation, depressed state, delirium, dementia, sleep disorder, anxiety disorder, social withdrawal and the like, in elderly person. Of these, loss of motivation, depressed state, delirium, dementia, anxiety disorder, and social withdrawal, in elderly person, are preferable targets of the agent for use in the prophylaxis or improvement of frailty of the present invention.

Prophylaxis of frailty is previous prevention of the above-mentioned symptoms from being developed, and improvement of frailty is a concept including bringing the above-mentioned symptoms to fall within a normal range, as well as preventing the progression (exacerbation) of the disease. The normal range here is determined based on the age, sex, height, basal metabolism, and amount of motor activity, for example.

Examples of the cause of frailty include physical factors such as low nutrition, sarcopenia, contraction of a chronic disease, chronic inflammation, smoking, decrease in sex hormone, social factors and environmental factors. Among these, the agent for use in the prophylaxis or improvement of frailty of the present invention is preferably used for frailty involving sarcopenia wherein muscle fiber number and muscle cross-sectional area decrease due to aging and physical functions are impaired by the overall loss of muscle mass.

The agent for use in the prophylaxis or improvement of frailty of the present invention can be applied to human, animals other than human [for example, mammals other than human (domestic animals and pet animals such as swine, bovine, horse and dog), birds (poultry and pet animals such as turkey, and chicken) etc.] in elderly generation.

The elderly generation for human means 65 to around 74 years old (early-stage elderly generation) and after 75 years old (late-stage elderly generation). In addition, the World Health Organization (WHO) in the United Nations defines the elderly person as those of age 65 or above.

The agent for use in the prophylaxis or improvement of frailty of the present invention means pharmaceutical products, quasi-drugs, health aid foods, foods with health claims, compositions similar to pharmaceutical products and having a particular function and ingested for the purpose of maintaining health such as supplement, and functional foods, which are used for the "prophylaxis or improvement of frailty" in elderly person.

In the agent for use in the prophylaxis or improvement of frailty of the present invention, the weight ratio of isoleucine and threonine is generally 1:0.2 - 10, preferably 1:0.3 - 8, more preferably 1:0.5 - 5.

When tryptophan is contained, the weight ratio of isoleucine, threonine and tryptophan is generally 1:0.2 - 10:0.06 - 8, preferably 1:0.3 - 8:0.1 - 5, more preferably 1:0.5 - 5:0.2 - 3.

When methionine is contained, the weight ratio of isoleucine, threonine and methionine is generally 1:0.2 - 10:0.1 - 5, preferably 1:0.3 - 8:0.2 - 4, more preferably 1:0.5 - 5:0.3 - 3.

In the agent for use in the prophylaxis or improvement of frailty of the present invention, the weight ratio of tryptophan, threonine, methionine and isoleucine is generally 1:0.5 - 12:0.2 - 10:0.5 - 12, preferably 1:1 - 9:0.5 - 8:1 - 9, more preferably 1:2 - 6:1 - 5:2 - 6.

The weight (%) of isoleucine or threonine in the free amino acid in the agent for use in the prophylaxis or improvement of frailty of the present invention is generally not less than 3%, preferably not less than 10%, more preferably not less than 20%, particularly preferably not less than 30%. Generally, that of isoleucine or threonine is not more than 97%, preferably not more than 90%, more preferably not more than 80%, particularly preferably not more than 75%, especially preferably not more than 70%.

When tryptophan is contained, the weight (%) of each amino acid in the free amino acid in the agent for the prophylaxis or improvement of frailty of the present invention is generally isoleucine 2.5% - 80%, threonine 2.5% - 80% and tryptophan 0.8% - 50%, preferably 8% - 75%, 8% - 75% and 3.5% - 40%, more preferably 20% - 70%, 20% - 70% and 5% - 33%, particularly preferably 30% - 70%, 30% - 70% and 12% - 33%.

When methionine is contained, the weight (%) of each amino acid in the free amino acid in the agent for use in the prophylaxis or improvement of frailty of the present invention is generally isoleucine 2.5% - 80%, threonine 2.5% - 80% and methionine 1.6% - 60%, preferably 8% - 75%, 8% - 75% and 6% - 50%, more preferably 20% - 70%, 20% - 70% and 15% - 45%, particularly preferably 30% - 70%, 30% - 70% and 22% - 45%.

When 4 kinds of amino acids are contained, the weight (%) of each amino acid in the contained amino acid (free amino acid) in the agent for use in the prophylaxis or improvement of frailty of the present invention is generally (1) tryptophan 0.5% - 45%, (2) threonine 1.5% - 70%, (3) methionine 1.0% - 50%, (4) isoleucine 1.5% - 70%, preferably (1) 3.5% - 40%, (2) 5% - 65%, (3) 6% - 45%, (4) 5% - 65%, more preferably (1) 5% - 33%, (2) 8% - 60%, (3) 10% - 40%, (4) 8% - 60%, further preferably (1) 10% - 33%, (2) 20% - 60%, (3) 15% - 40%, (4) 20% - 60%, particularly preferably (1) 15% - 33%, (2) 25% - 60%, (3) 22% - 40%, (4) 25% - 60%.

The contained amino acid means a free amino acid, and does not include constituent amino acid in protein or peptide.

While the dose of the agent for use in the prophylaxis or improvement of frailty of the present invention may vary depending on the age, sex, body weight, target disease, symptom, and dosage form, the daily dose of isoleucine and threonine in total is generally 40 mg - 15 g, preferably 67 mg - 13 g, more preferably 330 mg - 10 g, further preferably 330 mg - 4 g, particularly preferably 330 mg - 1.5 g, for an adult (e.g., body weight 60 kg), which is administered in once to several portions per day.

While the dose of the agent for use in the prophylaxis or improvement of frailty of the present invention may vary depending on the age, sex, body weight, target disease, symptom, and dosage form, the daily dose of isoleucine, threonine and tryptophan in total is generally 50 mg - 18 g, preferably 80 mg - 15 g, more preferably 400 mg - 12 g, further preferably 400 mg - 4 g, particularly preferably 400 mg - 1.6 g, for an adult (e.g., body weight 60 kg), which is administered in once to several portions per day.

While the dose of the agent for use in the prophylaxis or improvement of frailty of the present invention may vary depending on the age, sex, body weight, target disease, symptom, and dosage form, the daily dose of isoleucine, threonine and methionine in total is generally 50 mg - 20 g, preferably 90 mg - 18 g, more preferably 450 mg - 13 g, further preferably 450 mg - 4.5 g, particularly preferably 450 mg - 1.8 g, for an adult (e.g., body weight 60 kg), which is administered in once to several portions per day.

While the dose of the agent for use in the prophylaxis or improvement of frailty of the present invention may vary depending on the age, sex, body weight, target disease, symptom, and dosage form, the daily dose of amino acids (1) - (4) in total is generally 60 mg - 23 g, preferably 100 mg - 20 g, more preferably 500 mg - 15 g, further preferably 500 mg - 5 g, particularly preferably 500 mg - 2 g, for an adult (e.g., body weight 60 kg), which is administered in once to several portions per day.

The dose of (1) is generally 10 mg - 9 g, preferably 20 mg - 6 g, more preferably 80 mg - 5 g, particularly preferably 90 mg - 1 g, for an adult per day.

The dose of (2) is generally 30 mg - 20 g, preferably 60 mg - 18 g, more preferably 250 mg - 15 g, particularly preferably 250 mg - 1 g, for an adult per day.

The dose of (3) is generally 20 mg - 18 g, preferably 40 mg - 15 g, more preferably 170 mg - 12 g, particularly preferably 180 mg - 1 g, for an adult per day.

The dose of (4) is generally 30 mg - 20 g, preferably 60 mg - 18 g, more preferably 250 mg - 15 g, particularly preferably 250 mg - 1 g, for an adult per day.

The above-mentioned dose for an adult per day can be changed as appropriate in consideration of the sex, age, and condition of the body such as disease , for example.

The above-mentioned dose of the agent for use in the prophylaxis or improvement of frailty of the present invention can be administered all at once or in several portions. The dosing period is not particularly limited, and long-term administration is possible since the components are derived from amino acids.

The dosage form of the agent for use in the prophylaxis or improvement of frailty of the present invention is not particularly limited and either of oral preparation or parenteral preparation can be employed. Examples of the dosage form thereof include agents for oral administration such as tablet, granule, powder, capsule, elixir, syrup, microcapsule, drink, emulsion and suspension, skin external preparations such as ointment, cream, gel, liquid, lotion, facial mask and bathing powder, and injection.

For oral administration, the agent for use in the prophylaxis or improvement of frailty of the present invention can contain, where necessary, carrier, excipient, binder, swelling agent, lubricant, sweetening agent, flavor, preservative, emulsifier, and coating agent, and can be used together with these in a unit dosage form requested for the generally-acknowledged formulation and implementation. The amount of amino acid in these compositions or preparations only needs to be such amount that affords a suitable dose in the indicated range. In addition, the oral administration may be given any time before, after or between meals.

Examples of specific components that can be contained in the agent for use in the prophylaxis or improvement of frailty of the present invention include binders such as tragacanth, gum arabic, cornstarch and gelatin, polymeric polyvinylpyrrolidone excipients such as cellulose (e.g., microcrystalline cellulose, crystalline cellulose, hydroxypropylcellulose) and a derivative thereof; swelling agents such as cornstarch, pregelatinized starch, alginic acid, dextrin; lubricants such as magnesium stearate; flowability improvement agents such as fine silicon dioxide, methylcellulose; lubricants such as glycerol acid ester, talc, polyethylene glycol 6000 ; thickeners such as sodium carboxymethylcellulose, carboxyvinyl polymer, xanthan gum, gelatin ; sweetening agents such as sucrose, lactose and aspartam; flavors such as peppermint, vanilla flavor and cherry or orange; emulsifiers such as monoglyceride, polyglycerol ester of fatty acid, sucrose ester of fatty acid, lecithin, polyoxyethylenehydrogenated castor oil, polyoxyethylenemonostearic acid ester ; pH adjusters such as citric acid, sodium citrate, acetic acid, sodium acetate, sodium hydroxide ; flavoring agents such as aspartame, licorice extract, saccharin ; antioxidants such as erythorbic acid, butylhydroxyanisole, propyl gallate ; preservatives such as sodium benzoate, sodium edetate, sorbic acid, sodium sorbate, methyl parahydroxybenzoate, butyl p-hydroxybenzoate ; colorants such as red iron oxide, yellow ferric oxide, black ferric oxide, carmine, Food Color Blue No. 1, Food Color Yellow No. 4, Food Color Red No. 2 .

When the formulation unit form is a capsule, the above-mentioned types of materials can further contain a liquid carrier such as fats and oils.

Also, various other materials can be added to change the physical form of a formulation unit. Examples of the coating agent for tablet include shellac, sugar or both . Syrup or elixir can contain, for example, sucrose as a sweetening agent, methylparaben and propylparaben as preservative, dye and cherry or orange aroma.

Besides these, various vitamins and various amino acids may be added.

To provide an enteric preparation, for example, an aqueous solution of hydroxylphenylmethylcellulose is used as a coating pre-treatment agent, an aqueous solution of hydroxypropylmethylcellulosephthalate and an aqueous solution of polyacetin are used as coating agents and an enteric preparation is produced by a conventional method.

While the agent for use in the prophylaxis or improvement of frailty of the present invention contains at least isoleucine and threonine as active ingredients, an embodiment free of amino acids other than isoleucine and threonine, preferably, an embodiment free of amino acids other than isoleucine, threonine and tryptophan, or an embodiment free of amino acids other than isoleucine, threonine and methionine, can be mentioned, and an embodiment substantially free of amino acids other than (1) - (4) is more preferable. Being substantially free means that the object and effect of amino acids (1) - (4) are not influenced. The agent for use in the prophylaxis or improvement of frailty of the present invention may further contain any other active ingredients and other amino acids. As other amino acid, essential amino acid can be mentioned, and valine is preferable. The content of other amino acid is not more than 60 wt%, preferably not more than 40 wt%, further preferably not more than 20 wt%, of the amino acid contained.

The agent for use in the prophylaxis or improvement of frailty of the present invention may be used concurrently with other protein and peptide. The content of protein and peptide is preferably not more than 90 wt%, more preferably not more than 80 wt%, in consideration of a burden on the kidney of elderly person, and an embodiment substantially free of protein and peptide may also be used. Being substantially free means no influence on the object and effect of amino acids (1) - (4).

For parenteral administration, for example, a solution containing amino acids (1) - (4) can be nasally sprayed, or administered as an injection . When the agent for use in the prophylaxis or improvement of frailty of the present invention is used as a skin external preparation, amino acids (1) - (4) are dispersed in various bases, additives are added and the mixture is formulated according to a conventional method. Examples of such base and additive include higher fatty acid esters such as petrolatum, liquid paraffin, myristic acid isopropyl, myristic acid octyldodecyl ; higher alcohols such as squalane, lanolin, cetanol; fats and oils-base such as silicone oil, fats and oils from plant or animal ; lower alcohols such as ethanol; polyhydric alcohols such as polyethylene glycol, propylene glycol ; emulsion or emulsion stabilizer such as α-monoglycerylether, lecithin, sorbitan ester of fatty acid, dextrin fatty acid ester, fatty acid monoglyceride, fatty acid metal salt, magnesium sulfate ; aromatic, preservative, dye, thickener, antioxidant, UV protective agent, various medicinal agents such as wound healing agent, antiinflammatory agent, moisturizer and water .

In the present invention, a pharmaceutical product may be the agent for use in the prophylaxis or improvement of frailty of the present invention itself, or may contain other additives .

When the agent for use in the prophylaxis or improvement of frailty of the present invention is used as a food, it means a health food ingested by taking note of the particular function of the present invention, as well as a food for specified health uses and a food with nutrient function claims defined in the food with health claims, and also encompasses dietary supplements. The amount of amino acid contained in the food, which is eaten or drank per day, preferably falls within the same range mentioned above as that in the agent for use in the prophylaxis or improvement of frailty of the present invention. The form of a food with health claims as the agent for use in the prophylaxis or improvement of frailty of the present invention is not particularly limited.

While the food includes general food forms, for example, the agent for use in the prophylaxis or improvement of frailty of the present invention, and soup such as miso soup and corn soup, drinks such as green tea, coffee, tea and beverage, powder drinks, confectionery such as chocolate, cookies , ice cream, and jelly, which contain the agent and a suitable flavor, can be mentioned.

As the food in the present invention, a package form of a unit ingestion amount per serving of amino acid selected from (1) - (4) including at least isoleucine and threonine, a form of a drink of a suspension or solution of the aforementioned amino acid, which is filled in a bottle for a single serving can be mentioned. The dose per serving may be the above-mentioned daily dose.

Specifically, an agent for use in the prophylaxis or improvement of frailty in a unit package form per serving, which contains, in the unit, generally 4 mg - 15 g, preferably 7 mg - 13 g, further preferably 33 mg - 10 g, particularly preferably 33 mg - 4 g, of a total amount of isoleucine and threonine as an ingestion amount per serving can be mentioned. Particularly, an agent for use in the prophylaxis or improvement of frailty containing 33 mg - 1.5 g is preferable.

In addition, an agent for use in the prophylaxis or improvement of frailty in a unit package form per serving, which contains, in the unit, generally 5 mg - 18 g, preferably 8 mg - 15 g, further preferably 40 mg - 12 g, particularly preferably 40 mg - 4 g, of a total amount of isoleucine, threonine and tryptophan as an ingestion amount per serving can be mentioned. Particularly, an agent for use in the prophylaxis or improvement of frailty containing 40 mg - 1.6 g is preferable.

Furthermore, an agent for use in the prophylaxis or improvement of frailty in a unit package form per serving, which contains, in the unit, generally 5 mg - 20 g, preferably 9 mg - 18 g, further preferably 45 mg - 13 g, particularly preferably 45 mg - 4.5 g, of a total amount of isoleucine, threonine and methionine as an ingestion amount per serving can be mentioned. Particularly, an agent for use in the prophylaxis or improvement of frailty containing 45 mg - 1.8 g is preferable.

In addition, an agent for use in the prophylaxis or improvement of frailty in a unit package form per serving, which contains, in the unit, generally 6 mg - 23 g, preferably 10 mg - 20 g, further preferably 50 mg - 15 g, particularly preferably 60 mg - 10 g, of a total amount of amino acids (1) - (4) as an ingestion amount per serving can be mentioned. Particularly, an agent for use in the prophylaxis or improvement of frailty containing 60 mg - 2 g is preferable.

In addition, a food or drink for use in the prophylaxis or improvement of frailty in a unit package form per serving, which contains, in the unit, not less than 0.04 g of isoleucine and threonine in total, wherein the weight ratio of isoleucine and threonine is 1:0.2 - 10 is also one embodiment of the present invention. The above-mentioned total amount is preferably not less than 0.08 g, and preferably not less than 0.2 g. It is generally not more than 20 g, preferably1 not more than 12 g, more preferably not more than 8 g. The food and drink are similarly defined as the above-mentioned food.

Similarly, a food or drink for use in the prophylaxis or improvement of frailty in a unit package form per serving, which contains, in the unit, not less than 0.06 g of 4 kinds of amino acids of tryptophan, threonine, methionine and isoleucine in total, wherein the weight ratio of tryptophan, threonine, methionine and isoleucine is 1:0.5 - 12:0.2 - 10:0.5 - 12 is also one embodiment of the present invention. The above-mentioned total amount is preferably not less than 0.12 g, and preferably not less than 0.3 g. It is generally not more than 25 g, preferably1 not more than 18 g, more preferably not more than 12 g. The food and drink are similarly defined as the above-mentioned food.

Since a small amount of the aforementioned food or drink for use in the prophylaxis or improvement of frailty is effective, it is preferable for a dosage form such as tablet, granule, capsule , and can be conveniently ingested.

More concretely, a unit package form per serving, which contains, as an ingestion amount per serving in the unit, generally (1) 1 mg - 9 g, (2) 3 mg - 20 g, (3) 2 mg - 18 g, (4) 3 mg - 20 g, preferably (1) 2 mg - 6 g, (2) 6 mg - 18 g, (3) 4 mg - 15 g, (4) 6 mg - 18 g, more preferably (1) 8 mg - 5 g, (2) 25 mg - 15 g, (3) 17 mg - 12 g, (4) 25 mg - 15 g, can be mentioned. Therefore, when isoleucine and threonine are contained, and tryptophan and/or methionine are/is further contained, the above-mentioned amounts can be contained in combination in the unit package form.

In the case of a unit package form of 10 - 60 servings, the above-mentioned numerical values can be set to 10- to 60-fold.

In consideration of a food containing other amino acid, the weight (%) of each amino acid in the contained amino acids in the food or drink containing amino acids (1) - (4) of the present invention is
(1) tryptophan 0.5% - 45%, (2) threonine 1.5% - 70%, (3) methionine 1.0% - 50%, (4) isoleucine 1.5% - 70%, preferably (1) 3.5% - 40%, (2) 5% - 65%, (3) 6% - 45%, (4) 5% - 65%, further preferably (1) 5% - 33%, (2) 8% - 60%, (3) 10% - 40%, (4) 8% - 60%.

Specifically, a food or drink containing preferably not more than 40 wt%, further preferably not more than 20 wt%, of amino acid other than (1) - (4) of the contained amino acid is preferable.

Another embodiment of the present invention is a kit containing a measuring container, and a food or drink for use in the prophylaxis or improvement of frailty containing isoleucine and threonine as active ingredients.

The measuring container is not particularly limited as long as it is a container for measuring the amount of single use of the above-mentioned amino acids and, for example, a measuring cup and a measuring spoon can be mentioned. The amount of single use is the same as the amount described in the above-mentioned unit package form per serving and, for example, a total amount of not less than 0.04 g of isoleucine and threonine can be mentioned. The amount that can be measured by a measuring container can be determined according to the container such as a level amount or a heaping amount.

The measuring container may have a scale showing the amount of single use.

Another embodiment of the present invention is a kit containing a measuring container, and a food or drink for use in the prophylaxis or improvement of frailty containing 4 kinds of amino acids of tryptophan, threonine, methionine and isoleucine as active ingredients.

The measuring container is not particularly limited as long as it is a container for measuring the amount of single use of the above-mentioned amino acids and, for example, a measuring cup and a measuring spoon can be mentioned. The amount of single use is the same as the amount described in the above-mentioned unit package form per serving and, for example, a total amount of not less than 0.06 g of the 4 kinds of amino acids can be mentioned. The amount that can be measured by a measuring container can be determined according to the container such as a level amount or a heaping amount.

The measuring container may have a scale showing the amount of single use .

The present invention is explained in more detail in the following by referring to Examples, which are not to be construed as limitative of the scope of the present invention. In the present specification, % shows wt% unless particularly indicated.

### [Examples]

### [Example 1] granular powdered nutrition composition

In consideration of the effect on the prophylaxis of frailty, a granular powder nutrition composition was prepared based on the composition shown in Table 1. That is, a mixture having the composition of Table 1 was mixed in a compact high-speed mixer (NSK-150S, manufactured by Okadaseiko. co. jp) for 5 min. To the mixture were added distilled water and 99.5% alcohol at 2 - 5 wt%, and the mixture was kneaded in the mixer for 5 min to give a moistened kneaded mixture. The moistened kneaded mixture was granulated by an extrusion granulating machine with a 1.0 mmϕ screen, the obtained form was dried at a normal pressure and 70°C for 2 hr, sieved and 1.05 g of granules was filled in an aluminum bag. The whole components of the granular powder nutrition composition were stably maintained even one year later. In addition, the granular powder nutrition composition could be used by mixing with a nutritional supplement dissolved in warm water, and could be formed as an ion drink. Furthermore, it could be further mixed with a spice, salts such as sodium chloride, sodium glutamate, nucleic acid, and added to various foods.

**[Table 1]**

| | unit | wt% (weight ratio) |
|---|---|---|
| L-tryptophan | % | 10.46 (1.0) |
| L-threonine | % | 30.70 (2.9) |
| L-methionine | % | 21.01 (2.0) |
| L-isoleucine | % | 32.89 (3.1) |
| sucrose ester of fatty acid | % | 2.85 |
| flavor preparation | % | 0.95 |
| lecithin | % | 1.14 |
| 99.5% alcohol | | - |
| distilled water | | - |
| total | % | 100.0 |

### [Example 2] granular powdered nutrition composition

In the same manner as in Example 1 except that a mortar was used for kneading instead of the high-speed mixer, and a horizontal extruder (manufactured by Umetani Tekkosho) was used as the extrusion granulating machine, a granular powder could also be obtained.

### [Example 3] jelly nutrition composition

In consideration of the effect on the prophylaxis of frailty, a jelly nutrition composition was prepared as a nutrition composition based on the composition shown in Table 2. That is, to the composition of Table 2 were added a polysaccharide thickener and water, mixed, and emulsified by stirring with heating. After cooling, pH was adjusted to 3.8, and the mixture was sterilized by heating at 80°C for 10 min, 1.25 g was cooled and filled in a pouch. The whole components of the jelly nutrition composition were stably maintained even one year later.

**[Table 2]**

| | unit | wt% (weight ratio) | wt% (weight ratio) |
|---|---|---|---|
| L-tryptophan | % | 10 (1.0) | 10 (1.0) |
| L-threonine | % | 30 (3.0) | 41 (4.1) |
| L-methionine | % | 19 (1.9) | 10 (1.0) |
| L-isoleucine | % | 22 (2.2) | 20 (2.0) |
| lactose | % | 12 | 12 |
| sucrose | % | 7 | 7 |
| total | % | 100 | 100 |

### [Example 4] favorite drink powder

In careful consideration of the effect on the prophylaxis of frailty, powder preference drinks were prepared. The amounts of the starting materials to realize them are shown in Tables 3 - 6. Mixtures having the compositions of respective Tables were mixed by a high-speed mixer, and filled in aluminum bags. By changing the powder and flavor , Caramel Macchiato, café mocha, cocoa, and powdered green tea au lait could be produced similarly.

**[Table 3]**

| coffee | | |
|---|---|---|
| | unit | wt% (weight ratio) |
| L-tryptophan | % | 0.50 (1.0) |
| L-threonine | % | 1.60 (3.2) |
| L-methionine | % | 1.05 (2.1) |
| L-isoleucine | % | 1.60 (3.2) |
| sucrose | % | 41.55 |
| coffee whitener | % | 42.12 |
| coffee powder | % | 11.58 |
| total | % | 100 |

**[Table 4]**

| café au lait | | |
|---|---|---|
| | unit | wt% (weight ratio) |
| L-tryptophan | % | 0.34 (1.0) |
| L-threonine | % | 1.03 (3.0) |
| L-methionine | % | 0.68 (2.0) |
| L-isoleucine | % | 1.00 (2.9) |
| sucrose | % | 35.49 |
| coffee whitener | % | 23.27 |
| coffee powder | % | 5.82 |
| whey powder | % | 23.27 |
| defatted milk powder | % | 7.27 |
| sodium chloride | % | 0.29 |
| caramel dye | % | 0.39 |
| milk flavor | % | 0.62 |
| coffee flavor | % | 0.48 |
| xanthan gum | % | 0.05 |
| total | % | 100 |

**[Table 5]**

| green tea | | |
|---|---|---|
| | unit | wt% (weight ratio) |
| L-tryptophan | % | 5.05 (1.0) |
| L-threonine | % | 15.15 (3.0) |
| L-methionine | % | 10.10 (2.0) |
| L-isoleucine | % | 15.15 (3.0) |
| green tea powder | % | 16.36 |
| vitamin C | % | 1.82 |
| sodium hydrogen carbonate | % | 1.82 |
| maltodextrin | % | 25.64 |
| green tea flavor | % | 1. 64 |
| 0.25% aspartame | % | 7.27 |
| total | % | 100 |

**[Table 6]**

| milk tea | | |
|---|---|---|
| | unit | wt% (weight ratio) |
| L-tryptophan | % | 1.00 (1.0) |
| L-threonine | % | 2.00 (2.0) |
| L-methionine | % | 1.00 (1.0) |
| L-isoleucine | % | 2.00 (2.0) |
| sucrose | % | 61.75 |
| coffee whitener | % | 8.38 |
| tea powder | % | 1.61 |
| whey powder | % | 20.93 |
| sodium ascorbate | % | 0.63 |
| sucrose fatty acid ester | % | 0.31 |
| aspartame | % | 0.03 |
| milk flavor | % | 0.02 |
| tea flavor | % | 0.14 |
| emulsifier | % | 0.20 |
| total | % | 100 |

### [Example 5] powdered soup

In consideration of the effect on the prophylaxis of frailty, powdered soups were made based on the compositions shown in Tables 7 - 10. The starting materials shown in respective Tables were mixed by a high-speed mixer, and granulated in a fluidized bed. Furthermore, consomme soup and pumpkin soup could be produced similarly.

**[Table 7]**

| onion soup | | |
|---|---|---|
| | unit | wt% (weight ratio) |
| L-tryptophan | % | 2.00 (1.0) |
| L-threonine | % | 4.00 (2.0) |
| L-methionine | % | 3.00 (1.5) |
| L-isoleucine | % | 4.00 (2.0) |
| glucose | % | 20.75 |
| sucrose | % | 17.62 |
| sodium chloride | % | 25.87 |
| onion powder | % | 7.16 |
| garlic powder | % | 0.25 |
| potato starch | % | 7.34 |
| natural seasoning | % | 3.67 |
| white pepper powder | % | 0.21 |
| caramel dye | % | 0.37 |
| citric acid | % | 0.22 |
| consommé | % | 0.60 |
| *umami* seasoning | % | 2.93 |
| aspartame | % | 0.01 |
| total | % | 100 |

**[Table 8]**

| Chinese soup | | |
|---|---|---|
| | unit | wt% (weight ratio) |
| L-tryptophan | % | 3.00 (1.0) |
| L-threonine | % | 9.00 (3.0) |
| L-methionine | % | 6.00 (2.0) |
| L-isoleucine | % | 7.00 (2.3) |
| Chicken powder | % | 2.75 |
| chicken stock soup | % | 1.50 |
| chicken oil | % | 1.13 |
| sucrose | o % | 6.75 |
| sodium chloride | % | 28.50 |
| natural seasoning | % | 0.94 |
| *umami* seasoning | % | 15.60 |
| maltodextrin | % | 16.04 |
| chicken flavor | % | 1.58 |
| turmeric | % | 0.15 |
| white pepper powder | % | 0.03 |
| ginger powder | % | 0.03 |
| total | % | 100 |

**[Table 9]**

| corn soup | | |
|---|---|---|
| unit | unit | wt% (weight ratio) |
| L-tryptophan | % | 0.50 (1.0) |
| L-threonine | % | 1.00 (2.0) |
| L-methionine | % | 0.80 (1.6) |
| L-isoleucine | % | 1.00 (2.0) |
| Corn powder | % | 17.04 |
| cheese | % | 1.26 |
| powder cream | % | 16.85 |
| potato starch | % | 10.00 |
| sucrose | % | 22.70 |
| sodium chloride | % | 5.10 |
| onion powder | % | 5.26 |
| palm oil | % | 5.20 |
| whole milk powder | % | 5.02 |
| condensed milk | % | 3.80 |
| maltodextrin | % | 1.24 |
| guar gum | % | 1.05 |
| natural seasoning | % | 0.52 |
| *umami* seasoning | % | 0.32 |
| corn flavor | % | 1.20 |
| β-carotene | % | 0.08 |
| turmeric | % | 0.04 |
| aspartame | % | 0.02 |
| total | % | 100 |

**[Table 10]**

| miso soup | | |
|---|---|---|
| | unit | wt% (weight ratio) |
| L-tryptophan | % | 1.00 (1.0) |
| L-threonine | % | 3.30 (3.3) |
| L-methionine | % | 2.00 (2.0) |
| L-isoleucine | % | 3.00 (3.0) |
| miso powder | % | 57.91 |
| powder of dried bonito | % | 21.88 |
| bonito stock | % | 0.17 |
| sucrose | % | 3.45 |
| sodium chloride | % | 1.21 |
| natural seasoning | % | 1.79 |
| *umami* seasoning | % | 3.60 |
| citric acid | % | 0.69 |
| total | % | 100 |

### [Example 6] chocolate

In consideration of the effect on the prophylaxis of frailty, chocolate was prepared based on the composition shown in Table 11. Chocolate was dissolved in a water bath, and a flavor and amino acid were added and mixed well. The mixture was flown in a mold, and solidified by cooling in a refrigerator.

**[Table 11]**

| | unit | wt% (weight ratio) |
|---|---|---|
| L-tryptophan | % | 0.50 (1.0) |
| L-threonine | % | 2.00 (4.0) |
| L-methionine | % | 1.00 (2.0) |
| L-isoleucine | % | 1.50 (3.0) |
| black chocolate | % | 72.96 |
| white chocolate | % | 21.89 |
| chocolate flavor | % | 0.15 |
| total | % | 100 |

### [Example 7] cookie

In consideration of the effect on frailty, cookie was prepared based on the composition shown in Table 12. Sucrose was added to butter softened at room temperature, stirred well, and an egg was added and mixed. The remaining powder starting materials were added, and the mixture was stirred in a mixer, formed with a squeezer and baked in an oven at 165°C for 18 min.

**[Table 12]**

| | unit | wt% (weight ratio) |
|---|---|---|
| L-tryptophan | % | 0.20 (1.0) |
| L-threonine | % | 1.00 (5.0) |
| L-methionine | % | 0.50 (2.5) |
| L-isoleucine | % | 0.80 (4.0) |
| butter | % | 30.08 |
| sucrose | % | 16.50 |
| egg | % | 13.72 |
| sodium chloride | % | 0.26 |
| whole milk powder | % | 1.00 |
| soft wheat-flour | % | 35.90 |
| milk flavor | % | 0.04 |
| total | % | 100 |

### [Experimental Example 1] Consideration of effect of amino acid administration on elderly person at home

Based on the production method of Example 1, granules containing L-tryptophan, L-threonine, L-methionine and L-isoleucine at a weight ratio of 1:2.9:2.0:3.1 was produced. The granule corresponding to the total amount of 1 g of amino acid was ingested by 11 elderly persons (3 males, 8 females, average age 80.4±7.6) once per day for 2 months. Physical measurement was performed before and after the start of the amino acid granule ingestion, and grasping power, thigh circumference, and walking speed were measured.

In a seated position on a chair with both arms put down naturally on both sides of the body, a dynamometer (TTM yoken dynamo meter 50kg YCII senior & child) was held in a hand. The grasping range was adjusted to insure easy holding, and the subjects were instructed to grip the dynamometer as tight as possible. Measurement was performed once for right and left each at 0.1 kg unit, and the values thereof were recorded. In the analysis, the right and left were compared, and the maximum value was taken as the dominant hand and used for the test.

For statistical processing, an analysis software JMP9 was used, and the t-test of the related 2 groups was performed. The data are shown in mean±S.D., significant level was *p<0.05, and critical rate was 5%.

The results of the physical measurement before and after the start of the amino acid granule ingestion are shown in Table 13. As a result of the comparison of before and after the start of the amino acid granule ingestion, the grip strength and walking speed were significantly improved after the amino acid granule ingestion. Furthermore, the thigh circumference was maintained or improved for 2 months in 9 out of 11 subjects.

**[Table 13]**

| Results of physical measurement before and after amino acid granule ingestion | | | | |
|---|---|---|---|---|
| | before ingestion | 2 months after ingestion | | |
| | mean±S.D. | mean±S.D. | p value | number of subjects with improvement, maintenance |
| age (years old) | 80.4±7.6 | - | - | |
| grip strength (kg) | 15.0±4.1 | 17.9±5.4 | 0.025* | 8/11 |
| thigh circumference (cm) | 34.4±4.2 | 35.0±3.5 | 0.199 | 9/11 |
| walking speed (m/min) | 35.0±20.0 | 43.7±23.8 | 0.036* | 11/11 |

As a result of interview search by asking "Do you have any difficulty or distress at the moment?", 6 out of 11 subjects had some difficulty or distress before the start of the amino acid granule ingestion, whereas the number significantly decreased to 2 out of 11 subjects after the start of the amino acid granule ingestion.

### [Experimental Example 2] Consideration of effect of 4 kinds of amino acids on low protein model rat

### Test method

### (1) production of low protein model

Using 10-week-old CD(SD) male rats, they were raised on a 1 wt% casein feed obtained by reducing the protein in standard purified diet AIN-93G (manufactured by Oriental yeast Co., Ltd.) (Table 14). The animal fed on the 1 wt% casein diet for 28 days (4 weeks) was used as a low protein model, and an influence of amino acid administration was evaluated.

### (2) administration schedule

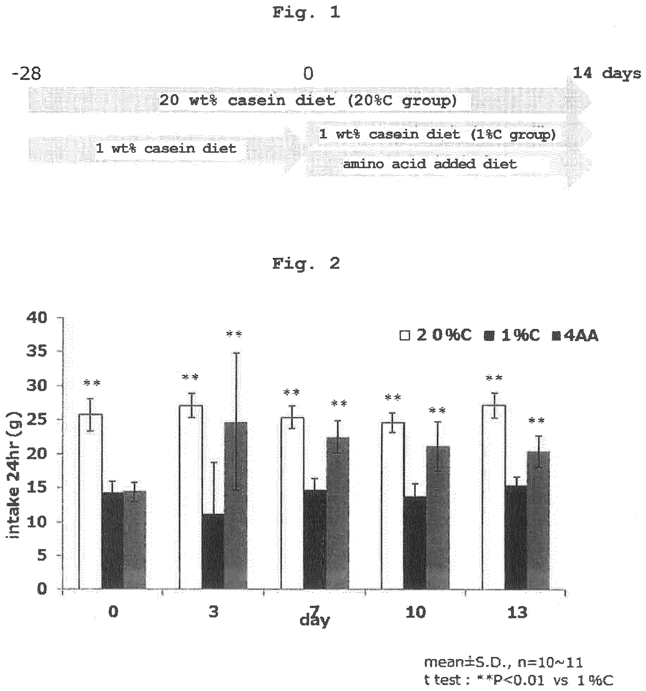

10-week-old CD(SD) male rats were divided into the groups shown in Table 15, casein 20 wt% (20%C group) or 1 wt% feed was fed for 28 days and the same feed was given thereafter to the 20%C group according to the schedule shown in Fig. 1. The low protein model rats were continuously given a 1 wt% casein feed obtained by reducing the protein in standard purified diet AIN-93G (1%C group) or a feed added with each amino acid shown in Table 16 (4AA group) for 2 weeks.

### (3) measurement of body weight and spontaneous activity

A daily feed intake was measured every day by using a balance system (Shimadzu Corporation) from the day when the amino acid added diet was given.

Spontaneous activity was measured by Low-Cost & MultiChannel System SUPERMEX (Muromachi Kikai. Co., Ltd.) utilizing a passive infrared sensor on the day before giving the amino acid added feed and in the dark period (12 hr) on day 12 from the feeding.

**[Table 14]**

| amount of amino acid (g) to be added per 1.0 kg feed (casein 10 g) | |
|---|---|
| Ile | 0.399 |
| Met | 0.254 |
| Thr | 0.371 |
| Trp | 0.127 |
| total | 1.151 |
| % | 0.115 |

**[Table 15]**

| group constitution | casein | addition amino acid | n |
|---|---|---|---|
| 20%C | 20 wt% | - | 10 |
| 1%C | 1 wt% | - | 11 |
| 4AA | 1 wt% | Ile/Met/Thr/Trp | 11 |

**[Table 16]**

| composition table (%) of test diet | | | | | | | | | |
|---|---|---|---|---|---|---|---|---|---|
| | 20% casein diet | 1% casein diet | | | | | | | |
| | 20%C | 1%C | 4AA | -Trp | -Thr | -Met | Ile,Thr | Ile | Thr |
| protein | | | | | | | | | |
| milk casein | 20 | 1 | 1 | 1 | 1 | 1 | 1 | 1 | 1 |
| L-cystine | 0.3 | 0.3 | 0.3 | 0.3 | 0.3 | 0.3 | 0.3 | 0.3 | 0.3 |
| Ile | | | 0.040 | 0.040 | 0.040 | 0.040 | 0.040 | 0.040 | |
| Met | | | 0.025 | 0.025 | 0.025 | | | | |
| Thr | | | 0.037 | 0.037 | | 0.037 | 0.037 | | 0.037 |
| Trp | | | 0.013 | | 0.013 | 0.013 | | | |

| hydrocarbonate | | | | | | | | | |
|---|---|---|---|---|---|---|---|---|---|
| cornstarch | 39.57 | 56.75 | 56.75 | 56.75 | 56.75 | 56.75 | 56.75 | 56.75 | 56.75 |
| α-cornstarch | 13.2 | 13.2 | 13.2 | 13.2 | 13.2 | 13.2 | 13.2 | 13.2 | 13.2 |
| granulated sugar | 10 | 10 | 10 | 10 | 10 | 10 | 10 | 10 | 10 |
| TK16 | | 2 | 2 | 2 | 2 | 2 | 2 | 2 | 2 |

| lipid | | | | | | | | | |
|---|---|---|---|---|---|---|---|---|---|
| purified soybean oil | 7 | 7 | 7 | 7 | 7 | 7 | 7 | 7 | 7 |
| cellulose powder | 5 | 5 | 5 | 5 | 5 | 5 | 5 | 5 | 5 |
| AIN-93VX | 1 | 1 | 1 | 1 | 1 | 1 | 1 | 1 | 1 |
| AIN-93G-MX | 3.5 | 3.5 | 3.5 | 3.5 | 3.5 | 3.5 | 3.5 | 3.5 | 3.5 |
| choline bitartrate | 0.25 | 0.25 | 0.25 | 0.25 | 0.25 | 0.25 | 0.25 | 0.25 | 0.25 |
| tertiary butylhydroquinone | 0.0014 | 0.0014 | 0.0014 | 0.0014 | 0.0014 | 0.0014 | 0.0014 | 0.0014 | 0.0014 |
| total | 100 | 100 | 100 | 100 | 100 | 100 | 100 | 100 | 100 |

### Results

The feed intake is shown in Fig. 2. In the 4AA addition free group, the feed intake decreased, but it was significantly improved by giving 4AA.

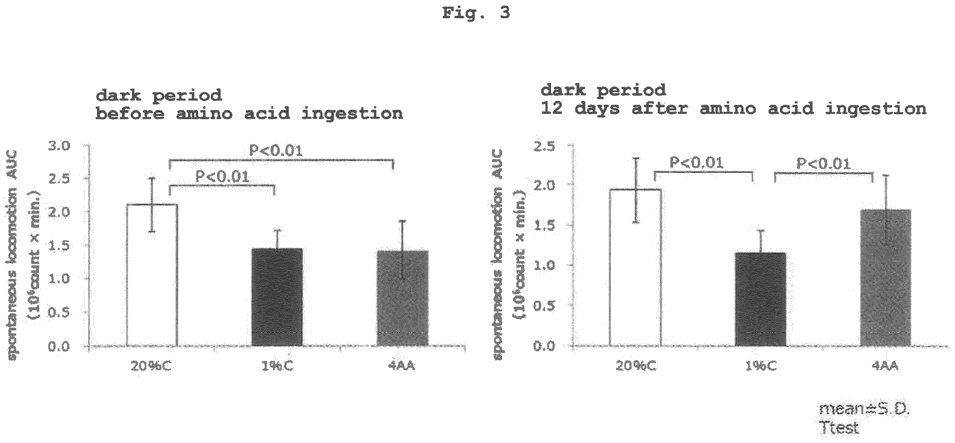

The spontaneous activity in the dark period significantly decreased in the 4AA addition free group compared to the control group, but it was significantly recovered by 4AA ingestion (remarkably observed particularly in the dark period). The results thereof are shown in Fig. 3.

### [Experimental Example 3] Consideration of influence of various amino acids on low protein model rat - 1

### Test method

10-week-old CD(SD) male rats were divided into the groups shown in Table 17, casein 20 wt% (20%C group) or 1 wt% feed was fed for 28 days according to the schedule shown in Fig. 1, and the body weight of rats thereafter fed with a 1 wt% casein diet (1%C group),and further added with each amino acid shown in Table 16 (4AA addition group (4AA group); a group of 4AA less tryptophan (-Trp group); a group of 4AA less methionine (-Met group); and a group of 4AA less threonine (-Thr group)) was measured.

**[Table 17]**

| group constitution | casein | amino acid added | n |
|---|---|---|---|
| 20%C | 20 wt% | - | 6 |
| 1%C | 1 wt% | - | 6 |
| 4AA | 1 wt% | Ile/Met/Thr/Trp | 6 |
| -Trp | 1 wt% | Ile/Met/Thr | 6 |
| -Thr | 1 wt% | Ile/Met/Trp | 6 |
| -Met | 1 wt% | Ile/Thr/Trp | 6 |

### results

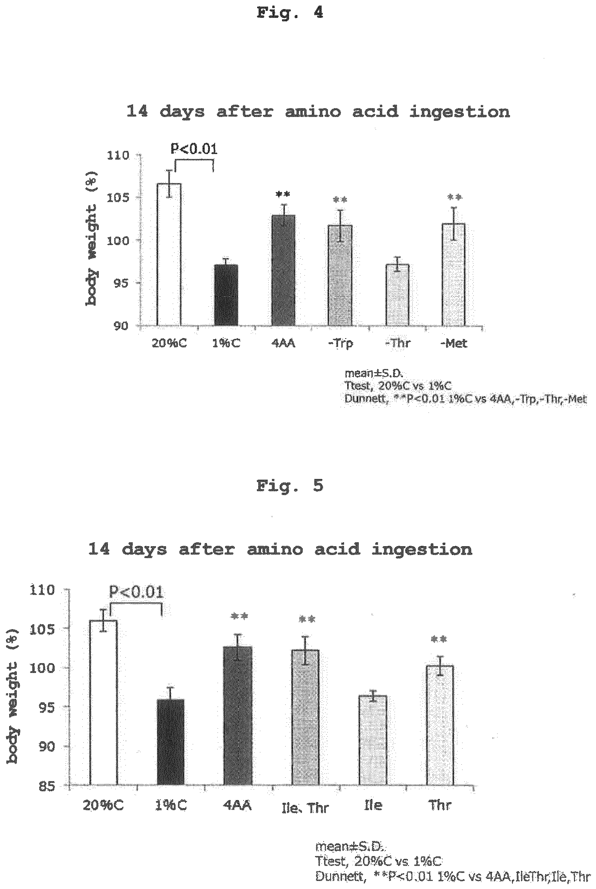

The body weight of the rats 14 days after feeding with the amino acid added diet was measured, the ratio (%) to the body weight measured on day 0 was calculated and shown in Fig. 4. In the 1%C group, the body weight decreased, whereas the body weight was significantly improved in the 4AA group, -Trp group and -Met group. In the -Thr group, the body weight was not improved.

### [Experimental Example 4] Consideration of influence of various amino acids on low protein model rat - 2

### Test method

10-week-old CD(SD) male rats were divided into the groups shown in Table 18, casein 20 wt% (20%C group) or 1 wt% feed was fed for 28 days according to the schedule shown in Fig. 1, and the body weight of rats thereafter fed with a 1 wt% casein diet (1%C group), and further added with the amino acid shown in Table 16 (4AA addition group (4AA group); an isoleucine and threonine combined use group (Ile, Thr group); an isoleucine addition group (Ile group); and the a threonine addition group (Thr group)) was measured.

**[Table 18]**

| group constitution | casein | amino acid added | n |
|---|---|---|---|
| 20%C | 20 wt% | - | 6 |
| 1%C | 1 wt% | - | 6 |
| 4AA | 1 wt% | Ile/Met/Thr/Trp | 6 |
| Ile, Thr | 1 wt% | Ile/Thr | 6 |
| Ile | 1 wt% | Ile | 6 |
| Thr | 1 wt% | Thr | 6 |

### Results

The body weight of the rats 14 days after feeding with the amino acid added diet was measured, the ratio (%) to the body weight measured on day 0 was calculated and shown in Fig. 5. In the 1%C group, the body weight decreased, whereas the body weight was significantly recovered in the 4AA group, Ile, Thr group, and Thr group. In the Ile group, increase of the body weight was weakened.

### [Experimental Example 5] Consideration of influence of other amino acids on low protein model rat

### Test method

10-week-old CD(SD) male rats were divided into the groups shown in Table 19, casein 20 wt% (20%C group) or 1 wt% feed was fed for 28 days according to the schedule shown in Fig. 1, and the body weight and weight of viscera-isolated carcass of rats thereafter fed for 14 days with a 1 wt% casein diet (1%C group), and further added with 1.15 g of the amino acid shown in Table 20 per 1 kg feed (4AA addition group (4AA group); a group of essential and non-essential amino acids addition group less 4AA (ALL-4AA group); essential amino acid addition group less 4AA (EAA-4AA group), non-essential amino acid addition group (NEAA group); and glycine addition group (Gly group)) was measured. The weight of viscera-isolated carcass to be the index of the weight of the skeletal muscle was obtained by measuring the body weight on day 14 from ingestion of each amino acid addition diet, exsanguinating the rat under anesthesia, isolating the viscera, and measuring the carcass.

**[Table 19]**

| group constitution | casein | amino acid added | n |
|---|---|---|---|
| 20%C | 20 wt% | - | 6 |
| 1%C | 1 wt% | - | 6 |
| 4AA | 1 wt% | Ile/Met/Thr/Trp | 6 |
| ALL-4AA | 1 wt% | ALL-4AA | 6 |
| EAA-4AA | 1 wt% | EAA-4AA | 6 |
| NEAA | 1 wt% | NEAA | 6 |
| Gly | 1 wt% | Gly | 6 |

**[Table 20]**

| composition table (%) of test diet | | | | | | | |
|---|---|---|---|---|---|---|---|
| | | 20% casein diet | | | | | |
| | | control | no addition | ALL-4AA | EAA-4AA | NEAA | Gly |
| protein | milk casein | 20 | 1 | | | | |
| | L-cystine | 0.3 | 0.3 | | | | |
| | essential, non-essential amino acid other than 4AA | | | 0.115 | | | |
| | essential amino acid other than 4AA | | | | 0.115 | | |
| | non-essential amino acid | | | | | 0.115 | |
| | L-glycine | | | | | | 0.115 |
| hydrocarbonate | cornstarch | 39.75 | 56.75 | 56.75 | 56.75 | 56.75 | 56.75 |
| | α-cornstarch | 13.2 | 13.2 | 13.2 | 13.2 | 13.2 | 13.2 |
| | granulated sugar | 10 | 10 | 10 | 10 | 10 | 10 |
| | TK16 | | 2 | 2 | 2 | 2 | 2 |
| lipid | purified soybean oil | 7 | 7 | 7 | 7 | 7 | 7 |
| | | | | | | | |
| | cellulose powder | 5 | 5 | 5 | 5 | 5 | 5 |
| | AIN-93VX | 1 | 1 | 1 | 1 | 1 | 1 |
| | AIN-93G-MX | 3.5 | 3.5 | 3.5 | 3.5 | 3.5 | 3.5 |
| | choline bitartrate | 0.25 | 0.25 | 0.25 | 0.25 | 0.25 | 0.25 |
| | tertiary butylhydroquinone | 0.0014 | 0.0014 | 0.0014 | 0.0014 | 0.0014 | 0.0014 |
| | total | 100 | 100 | 100 | 100 | 100 | 100 |

### Results

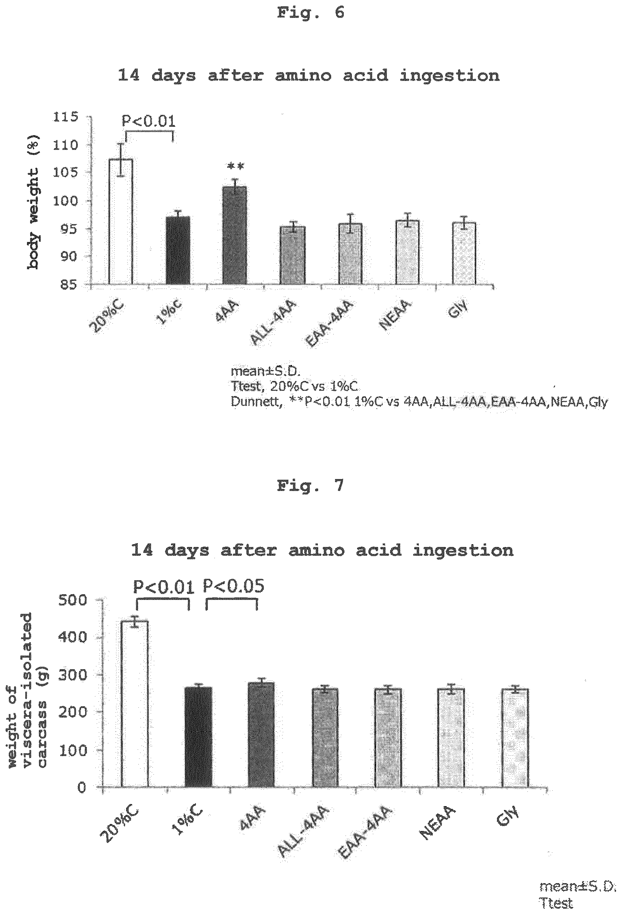

The body weight of the rats 14 days after feeding with the amino acid added diet was measured, the ratio (%) to the body weight measured on day 0 was calculated and shown in Fig. 6. In the 1%C group, the body weight decreased, whereas the body weight was significantly recovered in 4AA group. On the other hand, the body weight was not recovered in ALL-4AA group, EAA-4AA group, NEAA group and Gly group.

The weight of viscera-isolated carcass is shown in Fig. 7. The weight of viscera-isolated carcass significantly increased in 4AA group, and an increase in the muscle mass was observed.

The amino acid composition of the feeds used in Experimental Examples 2 - 5 (mass % to total free amino acid) and amino acid amount per 1 kg feed are shown in Table 21.

**[Table 21]**

| amino acid composition (wt% relative to total free amino acid) and amount of amino acid added per 1 kg feed | | | | | | | | | | | | | |
|---|---|---|---|---|---|---|---|---|---|---|---|---|---|
| | unit | TMIW | TMI | MIW | TIW | TI | I | T | ALL-TMIW | EAA - TMIW | NEAA | G | weight ratio |
| L-tryptophan | % | 11.0 | | 16.3 | 14.1 | | | | | | | | 1.0 |
| L-threonine | % | 32.3 | 36.3 | | 41.4 | 48.2 | | 100.0 | | | | | 2.9 |
| L-met hionine | % | 22.1 | 24.8 | 32.5 | | | | | | | | | 2.0 |
| L-isoleucine | % | 34.6 | 38.9 | 51.2 | 44.5 | 51.8 | 100.0 | | | | | | 3.1 |
| essential, non-essential amino acid other than TMIW | % | | | | | | | | 100.0 | | | | |
| essential amino acid other than TMIW | % | | | | | | | | | 100.0 | | | |
| non-essential amino acid | % | | | | | | | | | | 100.0 | | |
| L-glycine | % | | | | | | | | | | | 100.0 | |
| total amino acid amount | g/kg | 1.15 | 1.02 | 0.78 | 0.90 | 0.77 | 0.40 | 0.37 | 1.15 | 1.15 | 1.15 | 1.15 | |

In the Table, amino acid is indicated with a single letter, T: threonine, M: methionine, I: isoleucine, W: tryptophan, G: glycine. For example, TMIW shows a threonine, methionine, isoleucine and tryptophan added feed.

The ratio of essential and non-essential amino acids followed the general ratio of amino acid contained in casein.

## Claims

1. An agent for use in the prophylaxis or improvement of frailty, comprising isoleucine and threonine as active ingredients, wherein the content of amino acids other than tryptophan, threonine, methionine and isoleucine is not more than 60 wt% of the amino acid contained, and wherein the frailty is at least one kind of symptom selected from the group consisting of loss of muscle mass, decline in grip strength, feeling of fatigue, depression state, delirium, dementia, sleep disorder, anxiety disorder and social withdrawal, in an elderly person.

2. The agent for use according to claim 1, further comprising at least one kind of amino acid selected from tryptophan and methionine as an active ingredient.

3. The agent for use according to claim 1, wherein a weight ratio of tryptophan, threonine, methionine and isoleucine is 1:0.5 - 12:0.2 - 10:0.5 - 12.

4. The agent for use according to claim 2 or 3, wherein the weight (%) of tryptophan, threonine, methionine and isoleucine in the free amino acid in the agent is 5% - 33%, 8% - 60%, 10% - 40% and 8% - 60% respectively.

5. The agent for use according to any one of claims 1 to 4, which is in a unit package form per serving comprising not less than 0.06 g of an amino acid as an active ingredient in an ingestion amount per one serving.

6. The agent for use according to any one of claims 2 to 5, which is free of an amino acid other than tryptophan, threonine, methionine and isoleucine.

7. A food or drink for use in the prophylaxis or improvement of frailty, which is in a unit package form per serving comprising not less than 0.04 g of isoleucine and threonine in total as an active ingredient at a weight ratio of isoleucine and threonine of 1:0.2 - 10, wherein the content of amino acids other than tryptophan, threonine, methionine and isoleucine is not more than 60 wt% of the amino acid contained, and wherein the frailty is at least one kind of symptom selected from the group consisting of loss of muscle mass, decline in grip strength, feeling of fatigue, depression state, delirium, dementia, sleep disorder, anxiety disorder and social withdrawal, in an elderly person.

8. A food or drink for use in the prophylaxis or improvement of frailty according to claim 7, which is in a unit package form per serving comprising not less than 0.06 g of 4 kinds of amino acids of tryptophan, threonine, methionine and isoleucine in total as an active ingredient at a weight ratio of tryptophan, threonine, methionine and isoleucine of 1:2 - 6:1 - 5:2 - 6.

9. A kit comprising a measuring container and
a food or drink for use in the prophylaxis or improvement of frailty, comprising isoleucine and threonine as active ingredients, wherein the content of amino acids other than tryptophan, threonine, methionine and isoleucine in the food or drink is not more than 60 wt% of the amino acid contained, and wherein the measuring container is for measuring not less than 0.04 g in total of the aforementioned amino acid, wherein the frailty is at least one kind of symptom selected from the group consisting of loss of muscle mass, decline in grip strength, feeling of fatigue, depression state, delirium, dementia, sleep disorder, anxiety disorder and social withdrawal, in an elderly person.

10. A kit for use according to claim 9, comprising 4 kinds of amino acids of tryptophan, threonine, methionine and isoleucine as active ingredients, wherein the measuring container is for measuring not less than 0.06 g in total of the above-mentioned 4 kinds of amino acids.

11. Non-therapeutic use of an agent for the prophylaxis or improvement of frailty, comprising isoleucine and threonine as active ingredients, wherein the content of amino acids other than tryptophan, threonine, methionine and isoleucine is not more than 60 wt% of the amino acid contained, and wherein the frailty is at least one kind of symptom selected from the group consisting of decrease in walking speed, decrease in the amount of physical activity and loss of motivation, in an elderly person.

12. The non-therapeutic use according to claim 11, further comprising at least one kind of amino acid selected from tryptophan and methionine as an active ingredient.

13. The non-therapeutic use according to claim 11 or 12, wherein a weight ratio of tryptophan, threonine, methionine and isoleucine is 1:0.5 - 12:0.2 - 10:0.5 - 12.

14. The non-therapeutic use according to any one of claims 11 to 13, wherein the weight (%) of tryptophan, threonine, methionine and isoleucine in the free amino acid in the agent is 5% - 33%, 8% - 60%, 10% - 40% and 8% - 60% respectively.

15. The non-therapeutic use according to any one of claims 11 to 14, which is in a unit package form per serving comprising not less than 0.06 g of an amino acid as an active ingredient in an ingestion amount per one serving.

16. The non-therapeutic use according to any one of claims 11 to 15, which is free of an amino acid other than tryptophan, threonine, methionine and isoleucine.

17. Non-therapeutic use of a food or drink for the prophylaxis or improvement of frailty, which is in a unit package form per serving comprising not less than 0.04 g of isoleucine and threonine in total as an active ingredient at a weight ratio of isoleucine and threonine of 1:0.2 - 10, wherein the content of amino acids other than tryptophan, threonine, methionine and isoleucine is not more than 60 wt% of the amino acid contained, and wherein the frailty is at least one kind of symptom selected from the group consisting of decrease in walking speed, decrease in the amount of physical activity and loss of motivation, in elderly person.

18. The non-therapeutic use according to claim 17, which is in a unit package form per serving comprising not less than 0.06 g of 4 kinds of amino acids of tryptophan, threonine, methionine and isoleucine in total as an active ingredient at a weight ratio of tryptophan, threonine, methionine and isoleucine of 1:2 - 6:1 - 5:2 - 6.

## Patentansprüche

1. Mittel zur Verwendung bei der Prophylaxe oder Verbesserung von Gebrechlichkeit, umfassend Isoleucin und Threonin als aktive Bestandteile, wobei der Gehalt an anderen Aminosäuren als Tryptophan, Threonin, Methionin und Isoleucin nicht mehr als 60 Gew.-% der enthaltenen Aminosäure beträgt, und wobei die Gebrechlichkeit mindestens eine Art von Symptom ist, das aus der Gruppe ausgewählt ist, die aus Verlust von Muskelmasse, Rückgang der Griffstärke, Gefühl der Müdigkeit, depressivem Zustand, Delirium, Demenz, Schlafstörung, Angststörung und sozialem Rückzug bei einer älteren Person besteht.

2. Mittel zur Verwendung nach Anspruch 1, das ferner mindestens eine Art von unter Tryptophan und Methionin ausgewählte Aminosäure als Wirkstoff enthält.

3. Mittel zur Verwendung nach Anspruch 1, wobei das Gewichtsverhältnis von Tryptophan, Threonin, Methionin und Isoleucin 1:0,5 - 12:0,2 - 10:0,5 - 12 beträgt.

4. Mittel zur Verwendung nach Anspruch 2 oder 3, wobei das Gewicht (%) von Tryptophan, Threonin, Methionin und Isoleucin in der freien Aminosäure in dem Mittel 5 % - 33 %, 8 % - 60 %, 10 % - 40 % bzw. 8 % - 60 % beträgt.

5. Mittel zur Verwendung nach einem der Ansprüche 1 bis 4, das in einer Einheitspackungsform pro Portion vorliegt und nicht weniger als 0,06 g einer Aminosäure als Wirkstoff in einer Einnahmemenge pro Portion enthält.

6. Mittel zur Anwendung nach einem der Ansprüche 2 bis 5, das frei von einer anderen Aminosäure als Tryptophan, Threonin, Methionin und Isoleucin ist.

7. Nahrungsmittel oder Getränk zur Verwendung bei der Prophylaxe oder Verbesserung der Gebrechlichkeit, das in einer Einheitspackungsform pro Portion vorliegt, die insgesamt nicht weniger als 0,04 g Isoleucin und Threonin als Wirkstoff in einem Gewichtsverhältnis von Isoleucin und Threonin von 1:0,2 - 10 enthält, wobei der Gehalt an anderen Aminosäuren als Tryptophan, Threonin, Methionin und Isoleucin nicht mehr als 60 Gew.-% der enthaltenen Aminosäure beträgt, und wobei die Gebrechlichkeit mindestens eine Art von Symptom bei einer älteren Person ist, das aus der Gruppe ausgewählt ist, die aus Verlust von Muskelmasse, Abnahme der Griffstärke, Gefühl der Müdigkeit, Depressionszustand, Delirium, Demenz, Schlafstörung, Angststörung und sozialem Rückzug besteht.

8. Nahrungsmittel oder Getränk zur Verwendung bei der Prophylaxe oder Verbesserung der Gebrechlichkeit nach Anspruch 7, das in einer Verpackungseinheitsform pro Portion vorliegt, die nicht weniger als 0,06 g von insgesamt 4 Arten von Aminosäuren von Tryptophan, Threonin, Methionin und Isoleucin als Wirkstoff in einem Gewichtsverhältnis von Tryptophan, Threonin, Methionin und Isoleucin von 1:2 - 6:1 - 5:2 - 6 umfasst.

9. Kit, umfassend einen Messbehälter und ein Nahrungsmittel oder Getränk zur Verwendung bei der Prophylaxe oder Verbesserung der Gebrechlichkeit, umfassend Isoleucin und Threonin als aktive Bestandteile, wobei der Gehalt an anderen Aminosäuren als Tryptophan, Threonin, Methionin und Isoleucin in dem Nahrungsmittel oder Getränk nicht mehr als 60 Gew.-% der enthaltenen Aminosäure beträgt, und wobei der Messbehälter zur Messung von insgesamt nicht weniger als 0,04 g der oben genannten Aminosäure dient, wobei die Gebrechlichkeit mindestens eine Art von Symptom bei einer älteren Person ist, das aus der Gruppe ausgewählt ist, die aus Verlust von Muskelmasse, Abnahme der Griffstärke, Gefühl der Müdigkeit, Depressionszustand, Delirium, Demenz, Schlafstörung, Angststörung und sozialem Rückzug besteht.

10. Kit zur Verwendung nach Anspruch 9, umfassend 4 Arten von Aminosäuren von Tryptophan, Threonin, Methionin und Isoleucin als aktive Bestandteile, wobei der Messbehälter zur Messung von insgesamt nicht weniger als 0,06 g der oben genannten 4 Arten von Aminosäuren dient.

11. Nicht-therapeutische Verwendung eines Mittels zur Prophylaxe oder Verbesserung der Gebrechlichkeit, umfassend Isoleucin und Threonin als aktive Bestandteile, wobei der Gehalt an anderen Aminosäuren als Tryptophan, Threonin, Methionin und Isoleucin nicht mehr als 60 Gew.-% der enthaltenen Aminosäure beträgt, und wobei die Gebrechlichkeit mindestens eine Art von Symptom bei einer älteren Person ist, das aus der Gruppe ausgewählt ist, die aus Abnahme der Gehgeschwindigkeit, Abnahme des Ausmaßes der körperlichen Aktivität und Verlust der Motivation besteht.

12. Nicht-therapeutische Verwendung nach Anspruch 11, die ferner mindestens eine Art von Aminosäure, ausgewählt aus Tryptophan und Methionin, als Wirkstoff umfasst.

13. Nicht-therapeutische Verwendung nach Anspruch 11 oder 12, wobei das Gewichtsverhältnis von Tryptophan, Threonin, Methionin und Isoleucin 1:0,5 - 12:0,2 - 10:0,5 - 12 beträgt.

14. Nicht-therapeutische Verwendung nach einem der Ansprüche 11 bis 13, wobei das Gewicht (%) von Tryptophan, Threonin, Methionin und Isoleucin in der freien Aminosäure in dem Mittel 5 % - 33 %, 8 % - 60 %, 10 % - 40 % bzw. 8 % - 60 % beträgt.

15. Nicht-therapeutische Verwendung nach einem der Ansprüche 11 bis 14, die in Form einer Packungseinheit pro Portion vorliegt, die nicht weniger als 0,06 g einer Aminosäure als Wirkstoff in einer Einnahmemenge pro Portion enthält.

16. Nicht-therapeutische Verwendung nach einem der Ansprüche 11 bis 15, die frei von einer anderen Aminosäure als Tryptophan, Threonin, Methionin und Isoleucin ist.

17. Nicht-therapeutische Verwendung eines Lebensmittels oder Getränks zur Prophylaxe oder Verbesserung der Gebrechlichkeit, die in einer Einheitspackungsform pro Portion vorliegt, die nicht weniger als 0,04 g Isoleucin und Threonin insgesamt als aktiven Bestandteil in einem Gewichtsverhältnis von Isoleucin und Threonin von 1:0,2 bis 10 umfasst, wobei der Gehalt an anderen Aminosäuren als Tryptophan, Threonin, Methionin und Isoleucin nicht mehr als 60 Gew.-% der enthaltenen Aminosäure beträgt und wobei die Gebrechlichkeit mindestens eine Art von Symptom bei älteren Menschen besteht ist, das aus der Gruppe ausgewählt ist, die aus Abnahme der Gehgeschwindigkeit, Abnahme der körperlichen Aktivität und Motivationsverlust besteht.

18. Nicht-therapeutische Verwendung nach Anspruch 17, die in einer Einheitspackungsform pro Portion vorliegt, die nicht weniger als 0,06 g von insgesamt 4 Arten von Aminosäuren von Tryptophan, Threonin, Methionin und Isoleucin als Wirkstoff in einem Gewichtsverhältnis von Tryptophan, Threonin, Methionin und Isoleucin von 1:2 - 6:1 - 5:2 - 6 enthält.

## Revendications

1. Agent pour une utilisation dans la prophylaxie ou l'amélioration d'une faiblesse, comprenant de l'isoleucine et de la thréonine en tant que principes actifs, dans lequel la teneur en acides aminés autres que le tryptophane, la thréonine, la méthionine et l'isoleucine n'est pas supérieure à 60 % en poids des acides aminés contenus, et dans lequel la faiblesse est au moins un type de symptôme choisi dans le groupe constitué par une perte de masse musculaire, un déclin de la force de préhension, une sensation de fatigue, un état dépressif, un délire, une démence, un trouble du sommeil, un trouble de l'anxiété et un repli sur soi, chez une personne âgée.

2. Agent pour une utilisation selon la revendication 1, comprenant en outre au moins un type d'acide aminé choisi parmi le tryptophane et la méthionine en tant que principe actif.

3. Agent pour une utilisation selon la revendication 1, dans lequel le rapport en poids du tryptophane, de la thréonine, de la méthionine et de l'isoleucine est de 1 :0,5 - 12 :0,2 - 10 :0,5 - 12.

4. Agent pour une utilisation selon la revendication 2 ou 3, dans lequel le poids (%) de tryptophane, thréonine, méthionine et isoleucine parmi les acides aminés libres dans l'agent sont respectivement de 5 % à 33 %, 8 % à 60 %, 10 % à 40 % et 8 % à 60 %.

5. Agent pour une utilisation selon l'une quelconque des revendications 1 à 4, qui est sous une forme conditionnée unitaire par portion comprenant au moins 0,06 g d'un acide aminé, en tant que principe actif, dans la quantité ingérée par portion.

6. Agent pour une utilisation selon l'une quelconque des revendications 2 à 5, qui est exempt d'un acide aminé autre que le tryptophane, la thréonine, la méthionine et l'isoleucine.

7. Aliment ou boisson pour une utilisation dans la prophylaxie ou l'amélioration d'une faiblesse, qui est sous une forme conditionnée unitaire par portion comprenant au moins 0,04 g d'isoleucine et de thréonine au total, en tant que principes actifs, en un rapport en poids d'isoleucine et de thréonine de 1 :0,2 - 10, dans lequel la teneur en acides aminés autres que le tryptophane, la thréonine, la méthionine et l'isoleucine n'est pas supérieure à 60 % en poids des acides aminés contenus, et dans lequel la faiblesse est au moins un type de symptôme choisi dans le groupe constitué par une perte de masse musculaire, un déclin de la force de préhension, une sensation de fatigue, un état dépressif, un délire, une démence, un trouble du sommeil, un trouble de l'anxiété et un repli sur soi, chez une personne âgée.

8. Aliment ou boisson pour une utilisation dans la prophylaxie ou l'amélioration d'une faiblesse selon la revendication 7, qui est sous une forme conditionnée unitaire par portion comprenant au moins 0,06 g de 4 types d'acides aminés tryptophane, thréonine, méthionine et isoleucine au total, en tant que principes actifs, en un rapport en poids du tryptophane, de la thréonine, de la méthionine et de l'isoleucine de 1 :2 - 6 :1 - 5 :2 - 6.

9. Kit comprenant un récipient doseur et un aliment ou une boisson pour une utilisation dans la prophylaxie ou l'amélioration d'une faiblesse, comprenant de l'isoleucine et de la thréonine en tant que principes actifs, dans lequel la teneur en acides aminés autres que le tryptophane, la thréonine, la méthionine et l'isoleucine dans l'aliment ou la boisson n'est pas supérieure à 60 % en poids des acides aminés contenus, et dans lequel le récipient doseur est destiné à mesurer au moins 0,04 g au total des acides aminés susmentionnés, dans lequel la faiblesse est au moins un type de symptôme choisi dans le groupe constitué par une perte de masse musculaire, un déclin de la force de préhension, une sensation de fatigue, un état dépressif, un délire, une démence, un trouble du sommeil, un trouble de l'anxiété et un repli sur soi, chez une personne âgée.

10. Kit pour une utilisation selon la revendication 9, comprenant 4 types d'acides aminés tryptophane, thréonine, méthionine et isoleucine en tant que principes actifs, dans lequel le récipient doseur est destiné à mesurer au moins 0,06 g au total des 4 types susmentionnés d'acides aminés.

11. Utilisation non thérapeutique d'un agent pour la prophylaxie ou l'amélioration d'une faiblesse, comprenant de l'isoleucine et de la thréonine en tant que principes actifs, dans laquelle la teneur en acides aminés autres que le tryptophane, la thréonine, la méthionine et l'isoleucine n'est pas supérieure à 60 % en poids des acides aminés contenus, et dans laquelle la faiblesse est au moins un type de symptôme choisi dans le groupe constitué par une diminution de la vitesse de marche, une diminution de la quantité d'activité physique et une perte de motivation, chez une personne âgée.

12. Utilisation non thérapeutique selon la revendication 11, comprenant en outre au moins un type d'acide aminé choisi parmi le tryptophane et la méthionine en tant que principe actif.

13. Utilisation non thérapeutique selon la revendication 11 ou 12, dans laquelle le rapport en poids du tryptophane, de la thréonine, de la méthionine et de l'isoleucine est de 1 :0,5 - 12 :0,2 - 10 :0,5 - 12.

14. Utilisation non thérapeutique selon l'une quelconque des revendications 11 à 13, dans laquelle le poids (%) de tryptophane, thréonine, méthionine et isoleucine parmi les acides aminés libres dans l'agent sont respectivement de 5 % à 33 %, 8 % à 60 %, 10 % à 40 % et 8 % à 60 %.

15. Utilisation non thérapeutique selon l'une quelconque des revendications 11 à 14, qui est sous une forme conditionnée unitaire par portion comprenant au moins 0,06 g d'un acide aminé, en tant que principe actif, dans la quantité ingérée par portion.

16. Utilisation non thérapeutique selon l'une quelconque des revendications 11 à 15, qui est exempte d'un acide aminé autre que le tryptophane, la thréonine, la méthionine et l'isoleucine.

17. Utilisation non thérapeutique d'un aliment ou d'une boisson pour la prophylaxie ou l'amélioration d'une faiblesse, qui est sous une forme conditionnée unitaire par portion comprenant au moins 0,04 g d'isoleucine et de thréonine au total, en tant que principes actifs, en un rapport en poids d'isoleucine et de thréonine de 1 :0,2 - 10, dans laquelle la teneur en acides aminés autres que le tryptophane, la thréonine, la méthionine et l'isoleucine n'est pas supérieure à 60 % en poids des acides aminés contenus, et dans laquelle la faiblesse est au moins un type de symptôme choisi dans le groupe constitué par une diminution de la vitesse de marche, une diminution de la quantité d'activité physique et une perte de motivation, chez une personne âgée.

18. Utilisation non thérapeutique selon la revendication 17, qui est sous une forme conditionnée unitaire par portion comprenant au moins 0,06 g de 4 types d'acides aminés tryptophane, thréonine, méthionine et isoleucine au total, en tant que principes actifs, en un rapport en poids du tryptophane, de thréonine, de méthionine et d'isoleucine de 1 :2 - 6 :1 - 5 :2 - 6.
